# EUROPEAN PATENT APPLICATION

(11) **EP 1 854 496 A1**
(43) Date of publication of application: **14.11.2007**
(21) Application number: 05844829.1
(22) Date of filing: 28.12.2005
(51) Int. Cl.: A61M 25/00, A61M 31/00

(54) **BALLOON CATHETER**

(30) Priority: 28.02.2005 JP 2005054256
(71) Applicant: Nihon University, Tokyo 102-8275 (JP)
(72) Inventor: YOKOYAMA, Shinichiro, c/o Nihon University, Tokyo 102-8275 (JP); SAITO, Satoshi, c/o Nihon University, Tokyo 102-8275 (JP); FUKUDA, Noboru, c/o Nihon University, Tokyo 102-8275 (JP)
(74) Representative: Verhasselt, Jörn
(86) International application number: PCT/JP2005/024107
(87) International publication number: WO 2006/092903

(57) **Abstract**

A balloon catheter capable of adjusting a position to inflate a balloon in the axial direction of a catheter is provided.

This balloon catheter 1 comprises a catheter main body 2 formed with a plurality of lumina 5, 6, 7 extending along an axial direction in a lumen; an expansion body 3 surrounding the outer periphery of the catheter main body 2 and forming a sealed space with the outer peripheral surface of the catheter main body 2 and
formed by an elastic body expandable in the outer diameter direction by a fluid force-fed through a balloon lumen 6 from among a plurality of lumina; and sheath members (4a, 4c) surrounding the outer periphery of the expansion body 3 so as to partially constrict the expansion of the expansion body 3.

## Description

### BACKGROUND OF THE INVENTION

### Field of the Invention

The present invention relates to a balloon catheter.

### Description of the Related Art

For example, as a method of treatment for dilating a coronary artery narrowed by an obstructive system, there is known a percutaneous transluminal coronary angioplasty (PTCA) using a balloon catheter (hereinafter simply referred to also as catheter). The PTCA is a method of treatment, which allows the balloon catheter to be anchored in a lesion portion such as a stenosis portion and the like, and to inflate the balloon there, thereby dilating the stenosis portion.

Meanwhile, a practitioner of the PTCA is required to allow the balloon to be accurately anchored in each lesion portion. As a result, more the number of lesion portions to be treated are increased, more the burdens of the patent and the practioner are increased. Hence, for example, in case a plurality of lesion portions are in close vicinity, inflating a plurality of balloons formed in one catheter once so as to dilate a plurality of lesionportions at the same time is alsopracticed. However, a plurality of lesion portions are different in the location and the number of lesion portions are different according to the patents. Further, according to the conventional balloon catheter, the forming position of each balloon is fixed. Hence, the balloon catheters comprising the balloons of various sizes and lengths have been selected suitably by the patient or according to the purpose (for example, see JP2003-230629A).

### SUMMARY OF THE INVENTION

However, since the balloon catheter is selected suitably according to the patent or the purpose, the balloon catheter is built to a sort of order and becomes expensive. Further, the accurate position of the lesion portion cannot be decided simply by linear distance only, and depending on a bent state in the human organism, there is a case where fine adjustment of the balloon position is required. However, since a simply taking in and out of the catheter itself ends up totally moving a plurality of balloons, it is difficult to make proper adjustment so as to allow the balloon to be accurately anchored in each lesion portion.

The present invention has been carried out aiming at such problems, and an object of the invention is to provide a balloon catheter capable of adjusting a position where the balloon is inflated in the axial direction of the catheter.

To solve the above described problem, the invention according to claim 1 is a balloon catheter, comprising:
a catheter main body formed with a plurality of lumina expanded along an axial direction in a lumen;
a expansion body surrounding the outer periphery of the catheter main body so as to form a sealed space with the outer peripheral surface of the catheter main body and formed by an elastic body expandable in the outer diameter direction by a fluid force-fed through a balloon lumen from among said plurality of lumina; and
one or two more sheath members surrounding the outer periphery of the expansion body so that expansion of the expansion body is partially constricted.

Further, the invention according to claim2 is the catheter according to claim 1, wherein at least one of the plurality of lumina is a injection lumen for injecting medicine and the like,
wherein the expansion body has an adhesive portion adhered to the outer peripheral surface of the catheter main body in a portion possibly surrounded by the sheath portion, and moreover, the adhesive portion is comprised by being formed with a injection hole of the medicine and the like penetrating to the outside from the lumen of the injection lumen, and
wherein the sheath member surrounding the adhesive portion is passable with the medicine and the like, and is formed with a hole in a size not expandable by the expansion body.

Further, the invention according to claim 3 is the balloon catheter according to 1 or 2, wherein a plurality of sheath members are provided, and the plurality of sheath members are coupled by one or two more coupling portions having sheath members adjacent to each other.

Further, the invention according to claim 4 is the balloon catheter according to any one of claims 1 to 3, wherein the sheath members disposed at the position closest to said catheter main body and the base portion of the catheter main body have the screw portions mutually screwed into each other, respectively.

According to the present invention, the relative position between the catheter main body and the sheath member in the axial direction can be finely adjusted. Hence, the position
where the balloon is inflated in the axial direction of the catheter can be easily changed.

### BRIEF DESCRIPTION OF THE DRAWINGS

FIGS. 1A and 1B are views to explain a balloon catheter according to the embodiment of the present invention, and FIG. 1A is its longitudinal cross sectional view, and FIG. 1B is its side surface schematic diagram;
FIG. 2 is a cross sectional view cut along the line A-A in FIG. 1A;
FIG. 3 is a cross sectional view cut along the line B-B in FIG. 1A;
FIG. 4 is a cross sectional view cut along the line C-C in FIG. 1A;
FIGS. 5A to 5C are explanatory drawings on a balloon by the balloon catheter according to the embodiment of the present invention, FIG. 5A is a horizontal cross sectional view at a gap portion, and FIGS. 5B and 5C are view to explain the state of the formation of the balloon;
FIGS. 6A and 6B are side surface schematic diagrams to explain the balloon catheter according to the embodiment of the present invention, and illustrate that an expansion body is slightly expanded in the Figures;
FIG. 7 is a view to explain a usage example of the balloon catheter according to the embodiment of the present invention;
FIG. 8 is a cross sectional illustration in an using state of the balloon catheter according to the embodiment of the present invention;
FIG. 9 is a view to explain another example of the balloon catheter according to the present invention;
FIGS. 10A to 10C are views to explain an formation example of another balloon by the balloon catheter according to the embodiment of the present invention, and FIG. 10A is a longitudinal cross sectional view at the gap portion, and FIGS. 10B and 10C are views to explain a formation state of the balloon; and
FIGS. 11A to 11C are views to explain another example of the balloon catheter according to the present invention, and FIG. 11A is its longitudinal cross section view, and FIGS. 11B and 11C are side surface schematic diagrams, and illustrates that the expansion body 3 is in a slightly expanded state in FIGS. 11B and 11C.

### DETAILED DESCRIPTION OF THE PREFERRED EMBODIMENTS

One embodiment of the present invention will be described below with suitable reference to the drawings.

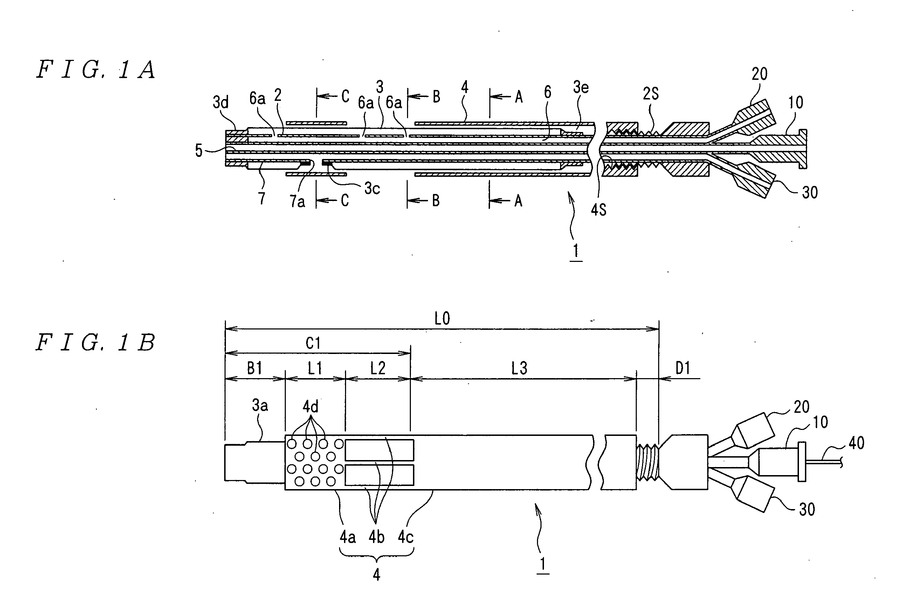

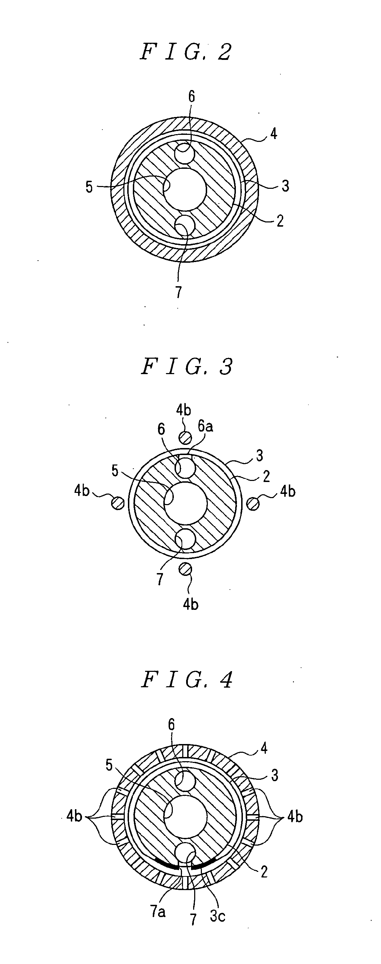

FIGS. 1A and 1B are explanatory drawings of a balloon catheter according to the present embodiment, and FIG. 1A is a longitudinal sectional viewof the balloon catheter according to the present embodiment, and FIG. 1B is a side surface schematic diagram. Further, FIG. 2 is a cross sectional view cut along the line A-A in FIG. 1A, FIG. 3 is a cross sectional view cut along the line B-B in FIG. 1A, and FIG. 4 is a cross sectional view cut along the line C-C in FIG. 1A.

This balloon catheter 1, as shown in FIG. 1A, is configured by comprising a catheter main body 2, an expansion body 3, and expansion constricting means 4.

The catheter main body 2 is a cylindrical body having flexibility, and is formed as three lumen configuration having three lumens extending along the axial direction. Further, the outer surface side of the base portion (right side in the Figure) is formed with a male screw portion 2s.

The three lumens (lumens), as shown in FIG. 2, are a guide lumen 5 which is the largest lumen formed approximately in the center, a balloon lumen 6 to expand the expansion body 3, and an injection lumen 7 to inject medicine and the like. The guide lumen 5, as shown in FIG. 1B is inserted with a guide wire 40 from a guide wire port 10. Further, the balloon lumen 6, as shown in FIGS.1A and 3, is formed with a plurality of fluid flowing holes 6a penetrating to the outside from the lumen by communicating with the inside of the expansion body 3. The balloon lumen 6 is force-fed with a specific fluid capable of controlling the pressure of the expansion body 3 from a balloon port 20. Further, the injection lumen 7, as shown in FIGS. 1A and 4, is formed with an injection hole 7a penetrating to the catheter main body 2 outside from the lumen by penetrating an adhesive portion 3c formed at one place in the expansion body 3. The medicine and the like can be force-fed from an injection port 30.

The expansionbody 3 uses an extremely thin rubber, silicon, and the like as a material, and is made of a raw material of uniform and excellent quality, and as shown in FIG. 1A, is attached to the top end side (the left side in the Figure) of the catheter main body 2. The expansion body 3 has both ends thereof (distal end 3d and proximal end 3e) in the axial direction fixed to the outer peripheral surface of the catheter main body 2, respectively, and surrounds the outer periphery of the catheter main body 2 and forms a sealed space with the outer peripheral surface of the catheter main body 2, thereby becoming an integral structure as a sort of one balloon. Further, the expansion body 3 has the adhesive portion 3c adhering a portion of the expansion body 3 and the outer peripheral surface of the catheter main body 2 at a predetermined position.

Here, the predetermined position is formed at a portion where a first sheath member 4a (to be described later) to supply necessary medicine and the like possibly surrounds the expansion body 3. In the present embodiment, at this predetermined position, the adhesive portion 3c is formed between two balloons formed by constriction by this first sheath member 4a. This adhesive portion 3c is formed with the injection hole 7a for the medicine and the like penetrating to the outside from the lumen of the injection lumen 7.

The expansion constricting means 4 is configured by comprising a plurality of sheath members surrounding the outer peripheries of the expansion body 3 and the catheter main body 2. A plurality of sheath members configuring the expansion constricting means 4 are formed by raw materials having flexibility, which partially constrict and overbear the expansion of the expansion body 3 from the outside of the expansion body 3.

To be more in detail, the expansion constricting means 4, as shown in FIG. 1B, is configured in order from the top end side (the left side in the Figure) by the first sheath member 4a, a coupling portion 4b, and a second sheath member 4c. An internal diameter size of each portion of the expansion restricting means 4 becomes a size surrounding at slight intervals the outer diameter of the expansion body 3 fixed to the catheter main body 2 and the outer peripheral surface of the catheter main body 2, and is movably loose fit in the axial direction of the catheter.

The first sheath member 4a, as shown in FIGS. 1B and 4, is formed as a portion of a predetermined distance L1 from the top end portion side of the expansion restricting means 4. The first sheath member 4a, as shown in FIG. 4, is a cylindrical member penetrating into the axial direction, and is provided with a medicine injection portion 4d comprising a number of minute through holes across the whole periphery. The size of a number of minute holes which become this medicine injection portion 4d is passable with necessary medicine and the like, and is formed with a size not expandable by the expansion body 3. This medicine injection portion 4d is formed at a position corresponding to the formation position of the injection hole 7a which penetrates to the outside from the lumen of the injection lumen 7. Here, though the corresponding position is desirable to be formed as a position opposing to the formation position of the injection hole 7a, since this balloon catheter 1 is configured such that the first sheath member 4a is movable in the axial direction, the corresponding position ends up moving within a minutely adjustable range. Hence, in the present embodiment, the medicine injection portion 4d is formed by the dozen across the whole periphery of the first sheath member 4a. The medicine injection portion 4d shown in FIGS. 1B and 4 illustrates an image where a number of holes are formed, and the holes actually formed are extremely minute.

The second sheath member 4c is formed by a raw material having flexibility, and as shown in FIG. 1B, is disposed at the position closest to the base portion of the catheter main body 2, and is a sheath-shaped cylindrical member of a distance L3 extending approximately to the base portion of the catheter main body 2. The second sheath member 4c is formed with a female screw portion 4s screwed into a male screw portion 2s in its internal surface side of the base portion. Hence, this balloon catheter 1 allows the mutual screw portions 2s and 4s of the catheter main body 2 and the expansion constricting means 4 (second sheath member 4c) to be screwed, thereby making it possible to prevent a shift in the axial direction when, for example, the catheter 1 is inserted into a body duct. Further, the base portion vicinity of the catheter main body 2 becomes a position not insertable into a body interior, and by taking in and out the whole expansion constricting means 4 in the axial direction by the mutual screw portions 2s and 4s outside the body, the position allowing the expansion body 3 to be expanded as a balloon can be minutely adjusted.

The coupling portion 4b, as shown in FIGS. 1B and 3, is a wire member having a narrow diameter, which couples the first sheathmember 4a and the second sheath member 4c of the expansion constricting means 4. This wire member uses a raw material smooth in the surface having a f lexibi lity and hard to be buckled. This coupling portion 4b is disposed at four places with equal intervals in the peripheral direction of the catheter. In this manner, this coupling portion 4b forms a gap portion (portion equivalent to a distance L2) between the adjacent first and second sheath members 4a and 4c, and this gap portion is divided into four places in the peripheral direction of the catheter mutually by the coupling portions 4b.

The whole length (L1+L2+L3) of the expansion restricting means 4, as shown in FIG. 1B, is shorter than the whole length L0 of the catheter main body 2. By the difference between these two lengths, as shown in the Figure, a balloon 3a at the top end side can be formed within the range of a distance B1, and an adjustment margin D1 by the mutual screw portions 2s and 4s can be secured.

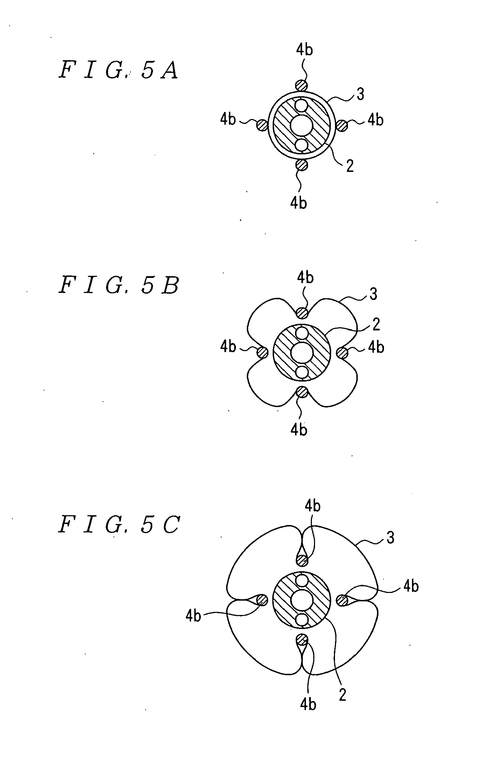

Each gap portion formed in the side surface of the expansion restricting means 4 permits an expansion of the expansion body 3 as a balloon within that gap portion. That is, as shown in FIGS. 5A to 5C, now, the pressure within the expansion body 3 is raised by force-feeding the fluid through the expansion balloon lumen 6 (FIG. 5A). Then, since the constriction of the expansion restricting means 4 does not operate at each of the gap portions, the expansion body 3 begins to expand from each gap portion (FIG. 5B). Here, the wire member forming the coupling portion 4b is narrow in diameter. That is, the width of the coupling portion 4b is set such that the expansion body 3 expanded from each of the divided gap portions can be mutually contacted when the expansion body 3 is expanded. Hence, by further raising the pressure, the expansion body 3 expanded from each of the divided gap portions is mutually contacted and becomes a size and a form to expand as one balloon across the whole periphery of the expansion restricting means 4 (FIG. 5C). In this manner, this balloon catheter 1 applies necessary strength and pressure on the fluid within the balloon lumen 6, so that the expansion body 3 is expanded from a portion not operated with the constriction of the expansion constricting means 4, and can be uniformly expanded and widened as a balloon.

Next, an example of how this balloon catheter is used will be described.

The following description is on how this balloon catheter is used for the treatment of a lesionportion (ischemic portion), that is, how this balloon catheter is used when the medicine, cells and the like are injected into the cardiac muscle system of the patient.

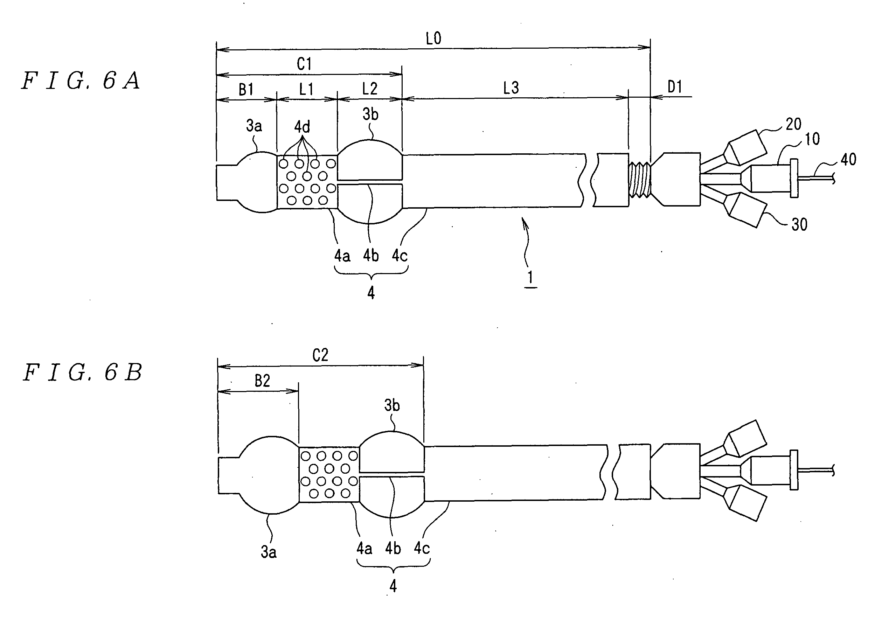

First, as preparation prior to the insertion of the balloon catheter 1, as shown in FIG. 6A, the position in the axial direction of the expansion restricting means 4 is adjusted, and initial positions of two balloons 3a and 3b are decided. For example, in the Figure, the position in the axial direction is decided by the screw portions 2s and 4s of each base portion so that the adjustment margin D1 is secured. At this time, the position of the base portion side of the balloon 3b becomes a distance C1.

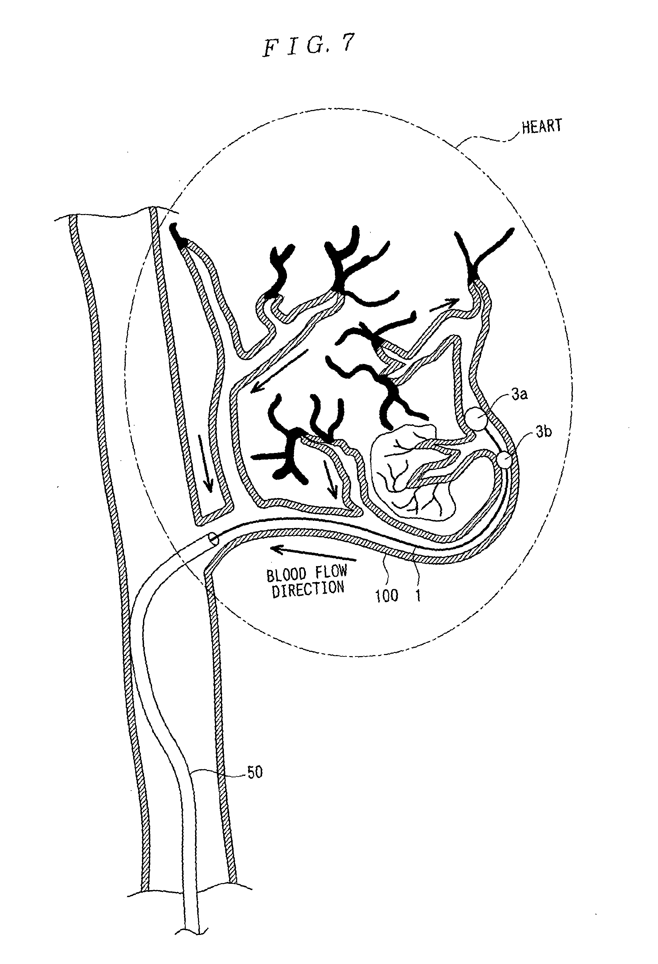

Next, as sown in FIG. 7, a guiding catheter 50 is inserted till the exit of a coronary vein 100, and is allowed to be anchored there.

Subsequently, the balloon catheter 1 according to the present invention in a state in which a guide wire 40 is built in advance is inserted into the coronary vein 100 in reverse to the blood flow by passing through the interior of the guiding catheter 50, and is adjusted so as to be anchored in the vascular region of the lesion portion periphery (where branched vessels connected to the lesion portion exist) between the two balloons of the balloon 3a of the top end side formed by inflating the expansion body 3 and the balloon 3b formed by inflating the expansion body 3 as shown FIGS. 5A to 5C. When the balloon catheter 1 is inserted, the expansion body 3 may be applied with pressurization so that the expansion body 3 is slightly expanded, for example, as shown in FIGS. 6A and 6B.

Here, for example, in case the position to anchor the balloon 3b is short of two to three mm of the expected initial position, the position in the axial direction of the expansion constricting means 4 is adjusted after the insertion by each of the screw portions 2s and 4s of each base portion, and as shown in FIG. 6B, the position of the base portion side of the balloon 3b can be changed to a distance C2.

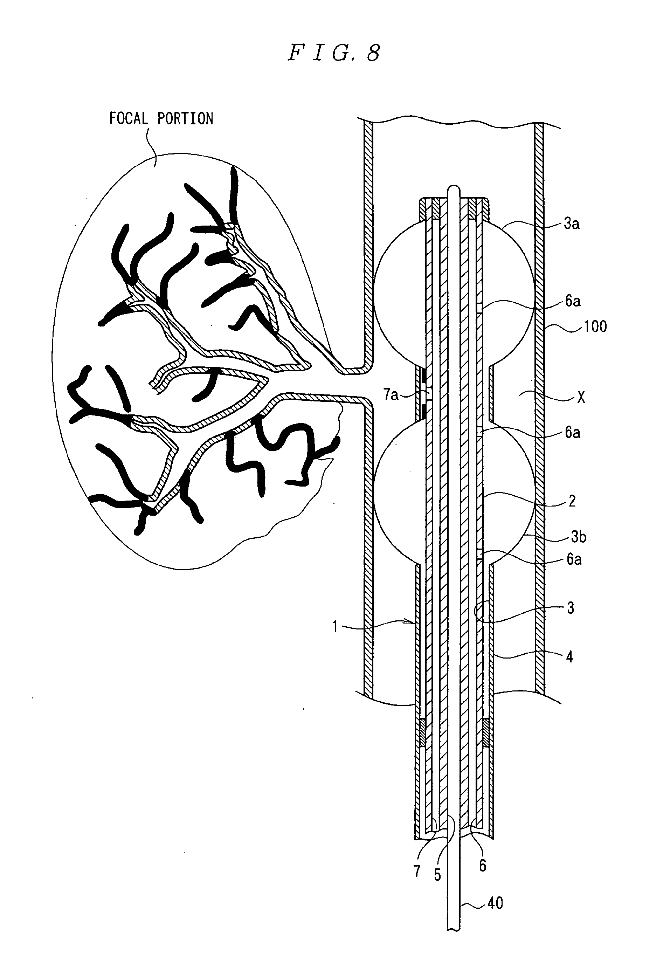

As shown in FIG. 8, the fluid is force-fed through the balloon lumen 6 to expand the expansion body 3, and each of the balloons 3a and 3b is inflated to partially obliterate the vessel, thereby forming an obliterated region X between both of the balloons 3a and 3b.

Next, the medicine and the like to be thrown into the lesion portion are force-fed from the injection port 30. The force-fed medicine and the like pass through the injection lumen 7, and are ejected from an inlet 7a with a predetermined pressure, and are supplied within the obliterated region X from a number of minute holes formed in the first sheath member 4a of the expansion constricting means 4 as the medicine injection portion 4d. Since the pressure within the obliterated region X is increased, the fluid within the obliterated region X is injected and dispensed to a focal portion by flowing backward within the branched veins branched within the obliterated region X. While, in FIG. 8, there is shown only one branched vein, it is a common practice to set one to ten branched veins to exist within the obliterated region X.

Next, the operation and advantages of this balloon catheter will be described.

This balloon catheter 1 comprises a flexible catheter main body 2 in which a plurality of lumens 5, 6, 7 extending along the axial direction are formed in its lumen. The expansion body 3 surrounding the outer periphery of the catheter main body 2 has the distal end and the proximal end fixed to the outer peripheral surface of the catheter main body 2, respectively, and is formed by an elastic body inflatable as a balloon in the outer radial direction of the catheter main body 2. This balloon catheter 1 further comprises flexible expansion constricting means 4 surrounding the outer periphery of the expansion body 3, and the expansion constricting means 4 has a gap portion between a plurality of sheath members, that is, between the first sheath member 4a and the second sheath member 4c which are adjacent to each other.

Here, the expansion body 3 is one-united body similarly as a sort of one balloon, and is partially expandable from the gap portion of the expansion restricting means 4 surrounding the outer periphery of the expansion body 3. The expansion restricting means 4, when the expansion body 3 is expanded, permits the expansion of the expansion body 3 as a balloon at the gap portion, and restricts the expansion of the expansion body 3 at other than the gap portion. In particular, the width of the coupling portion 4b is set such that the expansion body 3 expanded from each of the divided gap portions can be mutually contacted when the expansion body 3 is expanded. Hence, the gap portions of the expansion constricting means 4, as a result of mutual contact of the expansion body expanded from each gap portion divided by the coupling portion 4b, can be expanded as one balloon across the whole periphery of the expansion restricting means 4. The whole length of the expansion restricting means 4 is shorter than the whole length of the catheter main body 2, and by the difference of the length between both of them, the balloon 3s can be formed at the top end portion.

In this manner, this balloon catheter 1 forms the balloon 3a at the top end side by inflating one expansion body 3, and expands the expansion body 3 from each gap portion of the side surface of the expansion restricting means 4, and takes it asoneballoon3b, thereby a plurality of balloons can be formed.

Here, for example, usually, it is not possible to determine an accurate position of the focal portion and the like only by a linear distance. Hence, it is difficult to secure a matching adjustment margin even in case fine adjustment is required for the anchoring position of the balloon due to the bent in the body.

Hence, this balloon catheter 1 is configured such that the catheter main body 2 and the expansion constricting means 4 have the screw portions 2s and 4s mutually screwed at each base portion, and the adjustment margin D1 by both the screw portions 2s and 4s is secured. Further, the internal diameter size of each portion of the expansion constricting means 4 is a size surrounding with slight intervals the outer diameter size of the expansion body 3 fixed to the catheter main body 2 and the outer peripheral surface of the catheter main body 2, and is movably loose fit in the axial direction of the catheter.

According to such configuration, the expansion constricting means 4 only can be moved in the axial direction of the catheter. To be specific, in case the expansion constricting means 4 only is moved to the top end side of the catheter, as shown in FIG. 6A, the balloon 3a is inflated within the range of a distance B1 from the top end. Further, in case the expansion constricting means 4 only is moved to the base portion side, as shown FIG. 6B, the balloon 3a is inflated within the range of a distance B2 from the top end. By such an operation, the inflating position and size of the balloon 3a can be changed. At the same time, with respect to the balloon 3b, the position of the base portion side of the balloon 3b can be also changed from the distance C1 in FIG. 6A to the distance C2 in FIG. 6B.

In this manner, this balloon catheter 1 can arbitrarily decide a position at which the balloon is inflated within the range in which the screw portions 2s and 4s are screwed. Thus, the relative position between the catheter main body 2 and the expansion constricting means 4 can be easily fixed, and the fine adjustment of the relative position of the balloon can be also coped with. Consequently, for example, even in case the position at which the balloon should be anchored is short of two to three mm of the expected position as a result of the actual surgery operated on a specific patient, the position of the balloon can be finely adjusted if required. Usually, simply taking in and out of the catheter itself moves the whole catheter, and therefore, the fine adjustment similarly to the present invention cannot be performed.

Further, this balloon catheter 1, even in case the number of balloons or the interval between the balloons is changed, can cope with the situation by replacing the outside expansion constricting means 4 to meet the change. Hence, the catheter main body 2 and the expansion body 3 attached to the catheter main body 2 can be used as they are, and this is economical. That is, according to this catheter 1, since various sizes and lengths canbe coped with by one catheter, a balloon catheter having high versatility can be provided.

Further, this balloon catheter 1 comprises the injection lumen 7 for injecting the medicine and the like at least as one of a plurality of lumens. The expansion body 3, in one portion thereof, has an adhesive portion 3c adhered to the outer periphery of the catheter main body 2, and the adhesive portion 3c is formed with the injection hole 7a for the medicine and the like, which penetrates to the outside from the lumen of the injection lumen 7. Further, the expansion constricting means 4 is formed with the medicine injection portion 4d by the dozen, which is a hole of size capable of passing the medicine and the like and not inflatable by the expansion body 3. According to such configuration, for example, a locally obliterated region X, where exists a treatment site or branched veins, is formed by closing a vascular main duct by the balloons 3a and 3b, and the medicine and the like necessary for the treatment can be provided to a local treatment site from the medicine injection portion 4d.

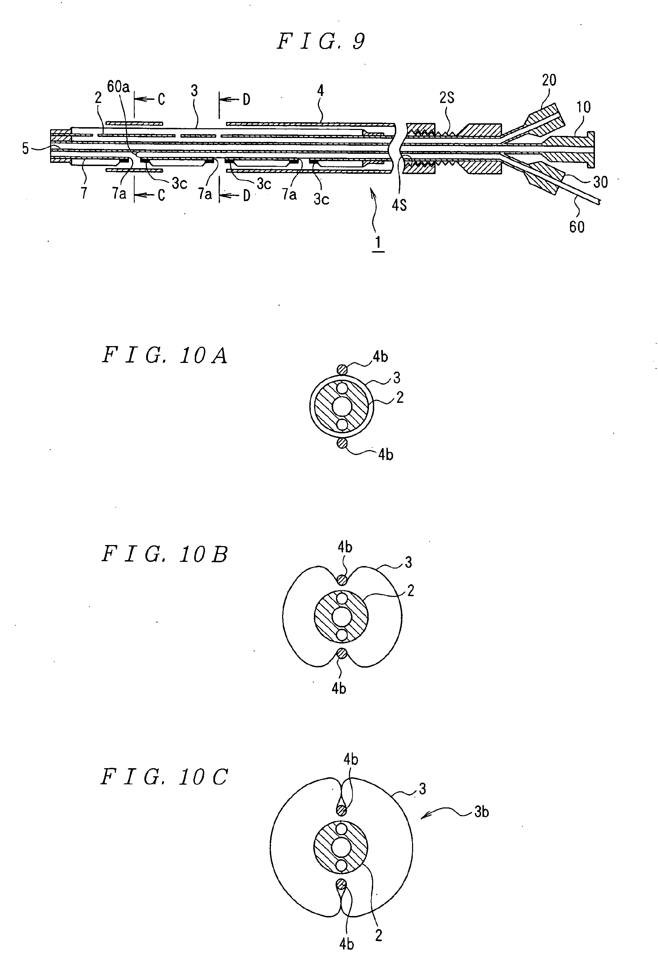

The balloon catheter according to the present invention is not limited to the above described embodiment, and various modifications are possible unless deviated from the spirit of the invention. For example, in the above described embodiment, though a description has been made on an example where the expansion body 3 is formed with the injection hole 7a for the medicine and the like on the adhesive portion 3c formed between two balloons 3a and 3b, the adhesive portion 3c and the injection hole 7a for the medicine and the like formed in the adhesive portion 3c are not necessary limited to the case of being formed between two balloons 3a and 3b, if the sheath portion to supply the medicine and the like is formed on a portion possibly surrounding the expansion body 3. For example, as a modified example of the above described embodiment, as shown by a longitudinal sectional view in FIG. 9, the adhesive portion 3c and the injection hole 7a for the medicine and the like formed in the adhesive portion 3c may be provided by the dozen in the axial direction of the balloon catheter 1. An injection tube 60 for throwing the medicine and the like is inserted from the injection portion 30 through the injection lumen 7. A top end portion 60a of the injection tube 60 is delivered to the injection hole 7a for the medicine and the like located at a desired position. Even according to such configuration, the medicine and the like can be force-fed similarly to the above described embodiment. If according to such configuration, the corresponding range of the adjustment of the gap portions of the first and second sheath portions as well as the injection position of the medicine and the like by replacement of the expansion constricting means 4 only can be expanded according to the positions and the number of the lesion portions, so that the versatility of the balloon catheter can be enhanced.

Further, for example, in the above described embodiment, while a description has been made on an example where a guide wire 40 is normally inserted into the lumen of the catheter 2, it is not limited to this, and even in case the guide wire is not used, the balloon catheter of the present invention can be applied.

Further, for example, in the above described embodiment, while a description has been made on an example where the space between the first and second sheath member 4a and 4c is coupled by the coupling portion 4b disposed at four places at equal intervals in the peripheral direction, it is not limited to this, and the space between the first and second sheath members 4a and 4c may be coupled by the coupling portion 4b disposed at two or three places. For example, in FIGS. 10A to 10C are shown a modified example in which the coupling portion 4b is disposed at two places. As shown in FIGS. 10A to 10C, even in this example similarly to the above described embodiment, the fluid within the balloon lumen 6 is applied with necessary pressure of strength, so that each of the expansion body 3 from the gap portions of a plurality of places of the expansion constricting means 4 can be uniformly expanded as one balloon.

Further, the balloon catheter according to the present invention is not necessarily limited to those coupling the space between the first and second sheath members 4a and 4c by the coupling portion. For example, as a modified example of the above described embodiment, the coupling portion 4b is not used, but the first sheath member 4a may be configured to be directly adhered to the expansion body 3. Even according to such configuration, by taking the space between the first and second sheath members 4a and 4c as the gap portion, the balloon 3b can be formed, and further, by moving the second sheath member 4c only from among the expansion constricting means 4 in the axial direction, the size and expansion length of the balloon 3b can be adjusted.

Further, for example, the number of balloons to be formed is not limited to two like the above described embodiment, it may be one or three mores. That is, since the number of sheath members configuring the expansion constricting means 4 and the positions of the mutual gap portions of the sheath members can be arbitrarily formed, the number of balloons can be easily increased. The size of the balloon can be also easily changed. Further, even in case the number of balloons is increased, since the expansion body 3 is one united-body, there is no need to increase the balloon lumen 6. Hence, even in case the number of balloon is many, an attempt at making the diameter of the catheter narrow can be made. The balloon lumen may be provided for every balloon individually.

Further, in the above described embodiment, while a description has been made on the case where the medicine and the like required for the local treatment portion only is supplied from the medicine injection portion 4d between two balloons 3a and 3b, the balloon catheter according to the present invention is not limited to such application.

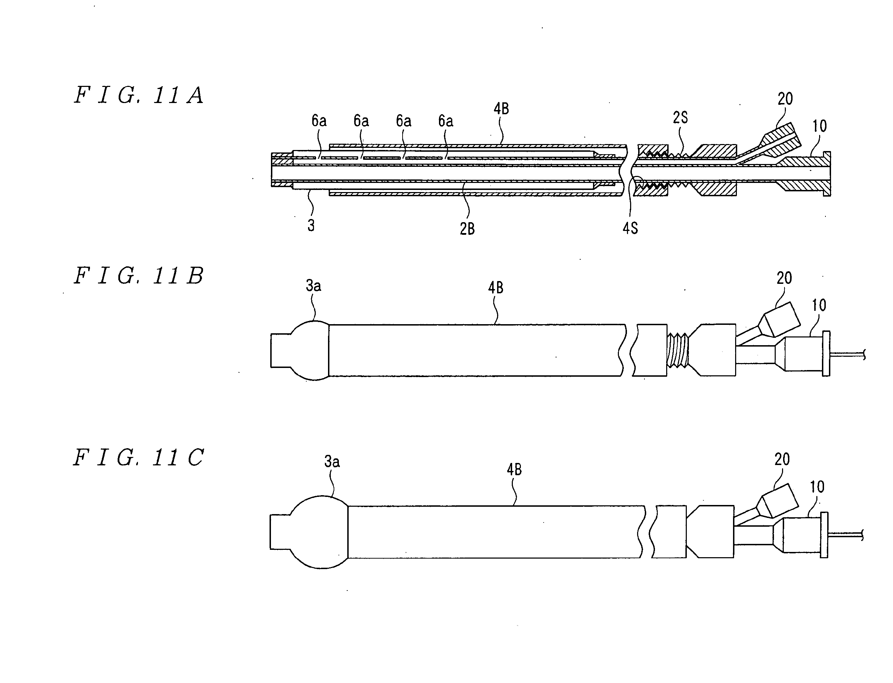

For example, in FIGS. 11A to 11C is shown another example of the balloon catheter according to the present invention. The example shown in the Figure, different from the above described embodiment, is not formed with the gap portion of the side surface and the medicine injection portion 4d in expansion constricting means 4. Further, it is different in that a catheter main body 2B is not provided with a medicine injection hole 7a, an injection lumen 7, and the like (see FIG. 11A). That is, according to this configuration, the expansion constrictingmeans 4B is not provided with a coupling portion, and becomes one sheath portion as a whole, and though the number of balloons to be formed is only one, similarly to the above described embodiment, the catheter main body 2B and the expansion constricting means 4B (sheath member) have screw portions 2s and 4s mutually screwed at each base portion. Hence, as shown in FIGS. 11B and 11C, the length of the catheter can be arbitrarily decided within the screwing range of screw portions 2s and 4s, and the relative position between the catheter 2B and the expansion constricting means 4B can be easily fixed, and the fine adjustment of the relative position can be coped with, and the size in the axial direction of the balloon 3a to be formed at the top end portion can be adjusted.

## Claims

1. A balloon catheter, comprising:
a catheter main body formed with a plurality of lumina expanded along an axial direction in a lumen;
a expansion body surrounding the outer periphery of the catheter main body so as to form a sealed space with the outer peripheral surface of the catheter main body and formed by an elastic body expandable in the outer diameter direction by a fluid force-fed through a balloon lumen from among said plurality of lumina; and
one or two more sheath members surrounding the outer periphery of the expansion body so that expansion of the expansion body is partially constricted.

2. The balloon catheter according to claim 1, wherein at least one of said plurality of lumina is an injection lumen for injecting medicine and the like,
wherein said expansion body has an adhesive portion adhered to the outer peripheral surface of said catheter main body in a portion possibly surrounded by said sheath portion, and moreover, the adhesive portion is comprised by being formed with a injection hole of the medicine and the like penetrating to the outside from the lumen of said injection lumen, and
wherein the sheath member surrounding the adhesive portion is passable with said medicine and the like, and is formed with a hole in a size not expandable by said expansion body.

3. The balloon catheter according to claim 1 or 2, wherein said plurality of sheath members are provided, and the plurality of sheath members are coupled by one or two more coupling portions having the sheath members adjacent to each other.

4. The balloon catheter according to any one of claims 1 to 3, wherein the sheath members disposed at the position closest to said catheter main body and the base portion of the catheter main body have the screw portions mutually screwed into each other, respectively.
